(19) Europäisches Patentamt
European Patent Office
Office européen des brevets

(11) Veröffentlichungsnummer: **0 196 574 B1**

(12) # EUROPÄISCHE PATENTSCHRIFT

(45) Veröffentlichungstag der Patentschrift: **10.10.90**

(51) Int. Cl.[5]: **C07C 217/82,** C07C 211/55

(21) Anmeldenummer: **86103879.2**

(22) Anmeldetag: **21.03.86**

(54) **4,4'-Diaminodiphenylverbindungen, Verfahren zu ihrer Herstellung und ihre Verwendung.**

(30) Priorität: **29.03.85 DE 3511545**

(43) Veröffentlichungstag der Anmeldung: **08.10.86 Patentblatt 86/41**

(45) Bekanntmachung des Hinweises auf die Patenterteilung: **10.10.90 Patentblatt 90/41**

(84) Benannte Vertragsstaaten: **CH DE FR GB IT LI**

(56) Entgegenhaltungen:
**CH-A- 598 328**
**US-A- 2 996 546**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80(DE)**

(72) Erfinder: **Hunger, Klaus, Dr., Johann-Strauss-Strasse 35, D-6233 Kelkheim (Taunus)(DE)**
Erfinder: **Frölich, Heinrich, Dr., Obernhäuser Weg 6, D-6272 Niedernhausen/Taunus(DE)**
Erfinder: **Habig, Kurt Conrad, Dr., Hundertmorgenring 98, D-6082 Mörfelden-Walldorf(DE)**

**EP 0 196 574 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 4,4'-Diaminodiphenylverbindungen die zur Herstellung von Farbstoffen und Pigmenten geeignet sind.

In der Stoffklasse der 4,4'-Diaminodiphenyle spielen Verbindungen, die in 3,3'-Stellung durch Methyl-, Methoxy-oder Ethoxygruppen substituiert sind, insbesondere als Bisdiazokomponenten für Farbstoffe, wie Direktfarbstoffe, Säurefarbstoffe, Lederfarbstoffe, Entwicklungsfarbstoffe, als Färbebasen, als Komponenten für Schwefelfarbstoffe und als Bisdiazokomponenten und in Kupplungskomponenten für Disazopigmente eine wichtige Rolle. Es wurden nun andere Verbindungen aus dieser Stoffklasse gefunden, die mit Vorteil zur Herstellung von neuen Farbstoffen und Pigmenten eingesetzt werden können.

Gegenstand der vorliegenden Erfindung sind 4,4'-Diaminodiphenylverbindungen der Formel I,

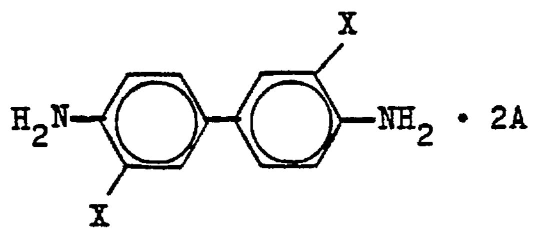

(I)

in der X den n-Propyl-, Isopropyl-, n-Butyl-, Isobutyl-, 1-Methylpropyl-, n-Propoxy-, Isopropoxy-, Isobutoxy-, 1- Methylpropoxy- oder 2-Methoxyethoxyrest bedeutet und A= O oder das Äquivalent einer anorganischen Säure ist.

Bevorzugt sind Verbindungen der Formel I, bei der die anorganische Säure Salzsäure oder Schwefelsäure ist.

Von besonderem Interesse sind Verbindungen der Formel I, bei der X den Isopropylrest bedeutet.

Von besonderem Interesse sind weiterhin die Verbindungen der Formel I, bei der X den n-Propoxy-, Isopropoxy-, Isobutoxy-, 1-Methylpropoxy- oder 2-Methoxyethoxyrest bedeutet.

Gegenstand der vorliegenden Erfindung ist auch eine Verfahren zur Herstellung einer Verbindung der allgemeinen Formel I, dadurch gekennzeichnet, daß man eine Verbindung der Formel II

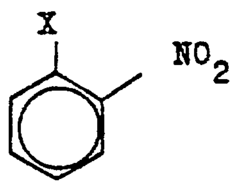

(II)

in der X die in der Verbindung der Formel I genannte Bedeutung hat, im alkalischen Medium zu der Verbindung der Formel III,

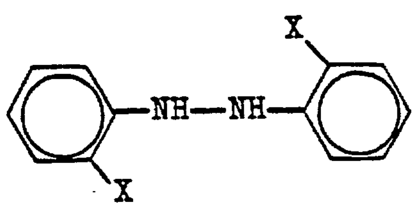

(III)

in der X die in der Verbindung der Formel I genannte Bedeutung hat, reduziert, anschließend die Verbindung der Formel III durch Behandlung mit einer Säure umlagert und das erhaltene Produkt in Form des freien Diamins, bzw. seines Salzes, der Formel I isoliert.

Die Reduktion der Nitrobenzolderivate der Formel II kann analog bekannten Reduktionsverfahren erfolgen, beispielsweise in wäßrig basischem Medium mit Zinkstaub, mit Natriumamalgam, mit Wasserstoff und Metallkatalysatoren, insbesondere Pd/C-Katalysatoren, oder elektrolytisch. Die Reduktion kann in homogener Lösung oder im Zweiphasensystem unter Zusatz von Lösungsvermittlern, wie Alkoholen oder Kohlenwasserstoffen, durchgeführt werden. Bevorzugt ist die Reduktion mit Zinkstaub in Natronlauge.

Neben dem gewünschten Hydrazobenzolderivat der Formel III tritt bei der Reduktion gewöhnlich als Nebenprodukt die der Nitroverbindung der Formel II entsprechende Aminover bindung auf. Das Nebenprodukt wird vorteilhaft vor der Weiterverarbeitung des Hydrazobenzolderivates abgetrennt, was meist in einfacher Weise durch Extraktion möglich ist.

Das erhaltene Hydrazobenzolderivat der Formel III wird anschließend im sauren Medium analog bekannten Methoden umgelagert. Als Mittel zur Umlagerung sind gewöhnlich starke anorganische Säuren in wäßriger oder wäßrig-alkoholischer Lösung geeignet. Auch Lösungen von Chlorwasserstoff in organischen Lösungsmitteln oder starke organische Säuren kommen in Frage. Von besonderem Interesse ist die Methode, bei der die Lösung der Hydrazoverbindung mit einem organischen Lösungsmittel wie Ether, Toluol, Chlorbenzol, Solventnaphtha oder dergleichen mit wäßrigen Mineralsäuren wie Halogenwasserstoffsäuren, Schwefelsäure, Phosphorsäure oder Salpetersäure verrührt wird.

Vorzugsweise wird das Hydrazobenzolderivat oder eine Lösung desselben in einem organischen Lösungsmittel mit Salzsäure oder wäßriger Schwefelsäure bei 5 bis 50°C, insbesondere bei 15 bis 30°C, behandelt. Die Reaktionszeit der Umlagerung hängt vom pH-Wert und dem jeweiligen Hydrazobenzolderivat ab und ist im mineralsauren Medium relativ am kürzesten.

Das Umlagerungsprodukt kann aus dem Rohproduktgemisch eventuell nach Reinigungsschritten, wie Filtration, Auswaschen mit organischen Lösungsmitteln, Umlösen oder Umkristallisieren in Form der 4,4'-Diaminodiphenylverbindung der Formel I, in dem A ein Rest der Säure aus der Umlagerungsreaktion ist, isoliert werden. Alternativ kann das Rohproduktgemisch, eventuell nach obengenannten Reinigungsschritten, neutralisiert werden und das Umlagerungsprodukt als freies Diamin der Formel (A= O) erhalten werden. In Einzelfällen ist es sinnvoll, das Umlagerungsprodukt in der Form des freien Diamins zu reinigen und dann mit der gleichen Säure wie bei der Umlagerung oder einer anderen geeigneten Säure zu Salzen der Formel I (A= anorganische Säure) umzusetzen und zu isolieren.

Gegenstand der Erfindung ist außerdem die Verwendung der Verbindungen der Formel I zur Herstellung von Farbmitteln.

Insbesondere sind die Verbindungen der Formel I zur Herstellung von Disazoverbindungen für Farbmittel geeignet, die durch Bisdiazotierung der erfindungsgemäßen Verbindungen und Umsetzung mit Kupplungskomponenten erhalten werden.

Als Farbmittel seien beispielsweise genannt: Farbstoffe, wie Direktfarbstoffe, anionische Farbstoffe (Säurefarbstoffe), Lederfarbstoffe, kationische Farbstoffe, Entwicklungsfarbstoffe oder Schwefelfarbstoffe, ferner Disazopigmente.

Für die Herstellung von Disazoverbindungen werden die erfindungsgemäßen Diamine nach bekannten Methoden, beispielsweise mit Natriumnitrit in wäßrig-salzsaurer Lösung oder mit Nitrosylschwefelsäure in schwefelsaurer Lösung oder in organischen Lösungsmitteln mit aliphatischen organischen Nitriten bisdiazotiert und anschließend mit zwei Äquivalenten einer Kupplungskomponente umgesetzt. Als Kupplungskomponenten sind beispielsweise Hydroxynaphthaline und alle sich davon ableitenden Verbindungen, wie Hydroxynaphthalinsulfonsäuren, Hydroxyaminonaphthalinsulfonsäuren, 2-Hydroxynaphthalin-3-carbonsäure, 2-Hydroxynaphthalin-3-carbonsäurearylide, aber auch methylenaktive Verbindungen, wie Acetessigsäurearylide oder Malonsäuredianilide und heterocyclische "ankuppelbare" Verbindungen, wie Barbitursäuren, 2,4-Dihydroxychinolin oder Pyridone, wie 4-Methyl-5-cyanpyridon-2, geeignet.

Die Verbindungen der Formel I eignen sich darüber hinaus als Bestandteil von bifunktionellen Kupplungskomponenten, die man beispielsweise durch Bisdiketenisierung der erfindungsgemäßen Diaminodiphenylderivate erhält und für Disazopigmente und -farbstoffe verwendet.

Die Verbindungen der Formel I sind aber auch zur Herstellung von Bisazomethin-Farbstoffen und -pigmenten geeignet, die beispielsweise durch Reaktion der Diamine mit Aldehyden erhalten werden. Zu erwähnen sind hier insbesondere aromatische und heterocyclische Aldehyde, beispielsweise der sogenannte Fischer-Aldehyd ((1,3,3-Trimethyl-2,3-dihydro-1-H-indol-2-yliden)acetaldehyd).

Weiter lassen sich die erfindungsgemäßen Verbindungen durch Reaktion mit aromatischen Kohlenwasserstoffen und Schwefel zu Schwefelfarbstoffen umsetzen.

Die Verbindungen der Formel I ergeben Färbebasen für die Naphtol-AS-Färberei mit verbesserter Naßechtheit und nach Diazotierung und Kupplung Disazo-Direktfarbstoffe auf Baumwolle mit zum Teil überraschend hoher Farbstärke.

Die folgenden Beispiele sollen die Erfindung näher beschreiben. Der Mutagenitätstest wurde nach Ames mit Salmonella typhimurium (Stämme TA 98, TA 100, TA 1535, TA 1537, TA 1538) ohne und in Gegenwart von S-9-Mix durchgeführt (B.N. Ames et al., Mut. Res. Vol. 31, S. 347 - 364 (1975)).

Beispiel 1

In einer Eisenapparatur mit Ankerrührer, Heiz- und Kühlvorrichtung werden 300 g o-Nitro-isopropylbenzol, 390 g Solventnaphta und 360 g Zinkstaub vorgelegt. Unter Rühren wird das Reaktionsgemisch auf ca. 65°C gebracht und zunächst 26 g 50 %ige Natronlauge, dann 23 g Wasser unter zeitweiser Kühlung zugegeben. Zur Vervollständigung der Reduktion wird bei einer Temperatur von etwa 70°C und Zugabe von weiterem Zinkstaub (60 g) nachgerührt, bis eine Tüpfelprobe mit der Reaktionslösung einen farblosen Auslauf zeigt. Durch einen weiteren Zusatz von Wasser wird dann das Zinkoxidhydrat gekörnt und abfiltriert. In dem organischen Filtrat befindet sich das 2,2'-Diisopropylhydrazobenzol sowie das als Nebenprodukt entstandene 1-Isopropyl-2-aminobenzol gelöst. Nach Extraktion des Nebenprodukts mit verdünnter Salzsäure läßt man das organische Filtrat in 40 %ige Schwefelsäure bei 15 bis 20°C zutropfen und rührt so lange nach, bis die Umlagerungsreaktion beendet ist (Probe: Keine Hydrazoverbindung mehr nachweisbar). Nach Neutralisation mit wäßrig-ammoniakalischer Lösung trennt man die heiße organische Phase ab und extrahiert mit verdünnter Salzsäure in der Hitze. Die Extraktionslösung wird mit Carboraffin geklärt und das Produkt aus dem salzsauren Filtrat unter Zugabe von konzentrierter Salzsäure abgeschieden. Man erhält 157 g 3,3-Diisopropyl-benzidin-dichlorhydrat mit einem Diazotierungswert von 99 %, Schmelzpunkt: 288°C. Die Verbindung ist im Mutagenitätstest nach Ames negativ.

Beispiel 2

In einem Eisenapparat mit Ankerrührer, Heiz- und Kühlvorrichtung werden 293 g o-Nitro-isobutoxybenzol, 395 g Solventnaphta und 220 g Zinkstaub vorgelegt. Unter Rühren wird das Reaktionsgemisch auf 80°C erhitzt und zunächst 24 g 50 %ige Natronlauge, dann 21 g Wasser unter zeitweiser Kühlung zugegeben. Zur Vervollständigung der Reduktion wird bei 80°C, unter portionsweiser Zugabe von weiterem Zinkstaub (insgesamt 53 g) nachgerührt, bis eine Tüpfelprobe mit der Reaktionslösung einen farblosen Auslauf zeigt. Durch einen weiteren Zusatz von Wasser wird dann das Zinkoxidhydrat gekörnt. Das Zinkoxidhydrat wird anschließend durch Filtration von der organischen Phase abgetrennt, in welcher das 2,2'-Diisobutoxy-hydrazobenzol sowie als Nebenprodukt das 1-Isobutoxy-2-aminobenzol enthalten ist. Nach Extraktion des Nebenprodukts mit verdünnter Salzsäure läßt man die organische Phase bei 15 - 20°C in Salzsäure tropfen und rührt bei 25°C so lange nach, bis die Umlagerungsreaktion beendet ist (Probe: Keine Hydrazoverbindung mehr nachweisbar). Die Reinigung des Rohprodukts erfolgt über Filtration, Auflösen in heißem Wasser und Klärung der Lösung mit Carboraffin. Nach Ausfällen mit konzentrierter Salzsäure erhält man 106 g 3,3'-Diisobutoxybenzidindichlorhydrat mit einem Diazotierungswert von 96 %, Schmelzpunkt 270°C.

Ein Teil des erhaltenen Produkts wird mit einer wäßrigen Ammoniaklösung neutralisiert und gelöst. Durch Extraktion in der Hitze und Kristallisation in der Kälte erhält man 3,3'-Di-isobutoxybenzidin mit einem Schmelzpunkt von 91°C. Die Verbindung ist im Mutagenitätstest nach Ames negativ.

Beispiel 3

In einem Eisenapparat mit Ankerrührer, Heiz- und Kühlvorrichtung werden 326 g o-Nitro-(2-methoxyethoxy)-benzol, 320 g Solventnaptha und 270 g Zinkstaub vorgelegt. Unter Rühren wird das Reaktionsgemisch auf ca. 70°C erhitzt und zunächst 34 g 35 %ige Natronlauge, dann 38 g Wasser unter zeitweiser Kühlung zugegeben. Zur Vervollständigung der Reduktion wird bei 70°C unter Zugabe von weiterem Zinkstaub nachgerührt, bis eine Tüpfelprobe mit der Reaktionslösung einen farblosen Auslauf zeigt. Durch einen weiteren Zusatz von Wasser wird die Körnung des Zinkoxidhydrats vorgenommen. Dieses wird darauf durch Filtration der organischen Phase abgetrennt, in welcher das 2,2'-Di-(2-methoxyethoxy)-hydrazobenzol sowie das als Nebenprodukt gebildete 1-(2-Methoxyethoxy)-2-aminobenzol enthalten ist. Nach Extraktion des Nebenprodukts mit verdünnter Salzsäure läßt man die organische Phase bei 12 bis 15°C zu 30 %iger Salzsäure tropfen und rührt bei 25°C so lange nach, bis die Umlagerungsreaktion beendet ist (Probe: Keine Hydrazoverbindung mehr nachweisbar). Anschließend wird mit wäßriger Ammoniaklösung neutralisiert und bei 85 bis 90°C die organische Phase abgetrennt. Durch Kaltrühren bringt man das 3,3'-Di-(2-methoxyethoxy)-benzidin zur Abscheidung. Nach Umkristallisieren aus Wasser erhält man 176 g Produkt mit einem Diazotierungswert von 98,8 %, Schmelzpunkt: 116°C. Die Verbindung ist im Mutagenitätstest nach Ames negativ.

In der folgenden Tabelle sind weitere Herstellungsbeispiele für Verbindungen der Formel I aufgeführt:

| Bsp. | X | A | Fp. [°C] | Ames-Test |
|---|---|---|---|---|
| 4 | $n-C_3H_7$ | HCl | 288 | negativ |
| 5 | $n-C_4H_9$ | HCl | 270 | negativ |
| 6 | $-CH_2CH(CH_3)_2$ | HCl | 258 | negativ |
| 7 | $-CH(CH_3)-CH_2-CH_3$ | HCl | 270 | negativ |
| 8 | $-n-OC_3H_7$ | HCl | 273 | negativ |
| 9 | $-OCH(CH_3)_2$ | HCl | 256 | negativ |
| 10 | $-O-CH(CH_3)CH_2CH_3$ | HCl | 240 | negativ |

**Patentansprüche**

1. Eine 4,4'-Diaminodiphenylverbindung der allgemeinen Formel I

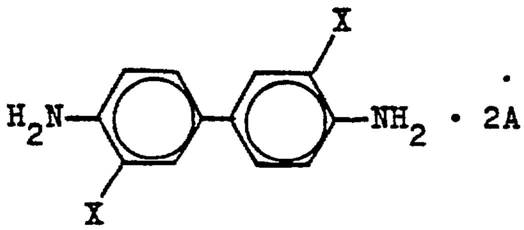

(I)

in der X den n-Propyl-, Isopropyl-, n-Butyl-, Isobutyl-, 1-Methylpropyl-, n-Propoxy-, Isopropoxy-, Isobutoxy-, 1-Methylpropoxy- oder 2-Methoxyethoxyrest bedeutet und A= O oder das Äquivalent einer anorganischen Säure ist.

2. Verbindung gemäß Anspruch 1, bei der die anorganische Säure Salzsäure oder Schwefelsäure ist.

3. Verbindung gemäß Anspruch 1 oder 2, dadurch gekennzeichnet, daß X den -Propyl-, Isopropyl-, n-Butyl-, Isobutyl- oder 1-Methylpropylrest bedeutet.

4. Verbindung gemäß Anspruch 1 oder 2, dadurch gekennzeichnet, daß X den n-Propoxy-, Isopropoxy-, Isobutoxy-, 1-Methylpropoxy oder 2-Methoxyethoxyrest bedeutet.

5. Verbindung gemäß Anspruch 1 oder 2, dadurch gekennzeichnet, daß X der Isopropylrest ist.

6. Verfahren zur Herstellung einer Verbindung der allgemeinen Formel I gemäß Anspruch 1, dadurch gekennzeichnet, daß man eine Verbindung der Formel II,

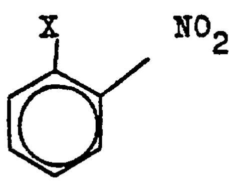

(II)

in der X die in der Verbindung der Formel I genannte Bedeutung hat, im alkalischen Medium zu der Verbindung der allgemeinen Formel III,

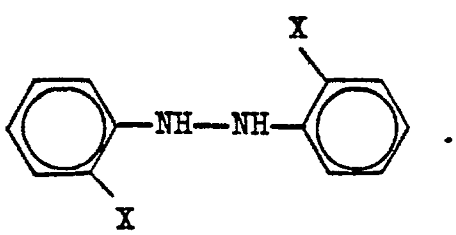

(III)

in der X die in Formel I genannte Bedeutung hat, reduziert, anschließend die Verbindung der Formel III durch die Behandlung mit einer Säure umlagert und das erhaltene Produkt in Form des freien Diamins, bzw. seines Salzes, der Formel I isoliert.

7. Verfahren gemäß Anspruch 6, dadurch gekennzeichnet, daß die Verbindung der Formel II mit Zinkstaub in Alkalilauge reduziert wird.

8. Verfahren gemäß Anspruch 6 oder 7, dadurch gekennzeichnet, daß die Verbindung der Formel III mit Salzsäure oder Schwefelsäure umgelagert wird.

9. Verfahren gemäß einem der Ansprüche 6 bis 8, dadurch gekennzeichnet, daß A in Formel I dem Äquivalent der Säure entspricht, mit der die Verbindung der Formel III umgelagert wird.

10. Verwendung der Verbindung gemäß Anspruch 1 zur Herstellung von Farbmitteln.

11. Verwendung gemäß Anspruch 10 zur Herstellung von Disazofarbstoffen oder Disazopigmenten.

12. Verwendung gemäß Anspruch 10 als Tetrazokomponente zur Herstellung von Disazo-Direktfarbstoffen.

13. Verwendung gemäß Anspruch 10 als Färbebase zur Herstellung von Entwicklungsfarbstoffen nach der Eisfarbentechnik.

**Claims**

1. A 4,4'-diaminobiphenyl compound of the general formula I

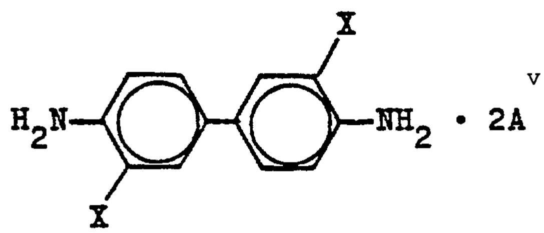

(I)

in which X denotes the n-propyl, isopropyl, n-butyl, isobutyl, 1-methylpropyl, n-propoxy, isopropoxy, isobutoxy, 1-methylpropoxy or 2-methoxyethoxy radical and A is ⊙ or one equivalent of an inorganic acid.

2. A compound as claimed in claim 1, wherein the inorganic acid is hydrochloric acid or sulfuric acid.

3. A compound as claimed in claim 1 or 2, wherein X denotes the propyl, isopropyl, n-butyl, isobutyl or 1-methylpropyl radical.

4. A compound as claimed in claim 1 or 2, wherein X denotes the n-propoxy, isopropoxy, isobutoxy, 1-methylpropoxy or 2-methoxyethox radical.

5. A compound as claimed in claim 1 or 2, wherein X is the isopropyl radical.

6. A process for preparing a compound of the general formula I as claimed in claim 1, which comprises reducing a compound of the formula II

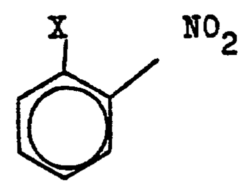

(II)

in which X has the meaning mentioned in the compound of the formula I, in an alkaline medium to the compound of the general formula III

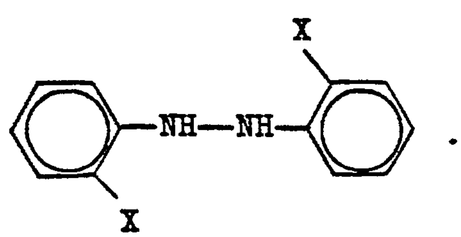

(III)

in which X has the meaning mentioned in the formula I, then rearranging the compound of the formula III by treatment with an acid and isolating the resulting product in the form of the free diamine, or its salt, of the formula I.

7. The process as claimed in claim 6, wherein the compound of the formula II is reduced with zinc dust in an alkali metal hydroxide solution.

8. The process as claimed in claim 6 or 7, wherein the compound of the formula III is rearranged with hydrochloric acid or sulfuric acid.

9. The process as claimed in any one of claims 6 to 8, wherein A in the formula I corresponds to one equivalent of the acid with which the compound of the formula III is rearranged.

10. Use of the compound as claimed in claim 1, for preparing colorants.

11. Use as claimed in claim 10, for preparing disazo dyes or disazo pigments.

12. Use as claimed in claim 10, as tetrazo component for preparing disazo direct dyes.

13. Use as claimed in claim 10, as dye base for preparing azoic dyes by ice color technique.

**Revendications**

1. Composé du diamino-4,4′ biphényle répondant à la formule générale I:

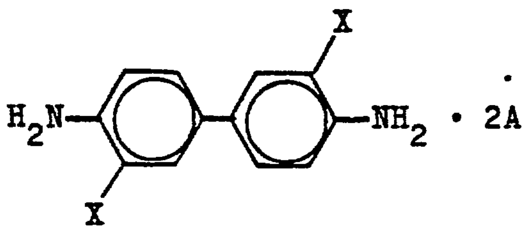

(I)

dans laquelle X représente un radical n-propyle, isopropyle, n-butyle, isobutyle, méthyl-1 propyle, n-propoxy, isopropoxy, isobutoxy, méthyl-1 propoxy ou méthoxy-2 éthoxy, et A ne représente rien ou représente l'équivalent d'un acide minéral.

2. Composé selon la revendication 1 dans lequel l'acide minéral est l'acide chlorhydrique ou l'acide sulfurique.

3. Composé selon l'une des revendications 1 et 2 caractérisé en ce que X représente un radical propyle, isopropyle, n-butyle, isobutyle ou méthyl-1 propyle.

4. Composé selon l'une des revendications 1 et 2 caractérisé en ce que X représente un radical n-propoxy, isopropoxy, isobutoxy, méthyl-1 propoxy ou méthoxy-2 éthoxy.

5. Composé selon l'une des revendications 1 et 2 caractérisé en ce que X représente un radical isopropyle.

6. Procédé pour préparer un composé de formule générale I selon la revendication 1, procédé caractérisé en ce qu'on réduit un composé répondant à la formule II:

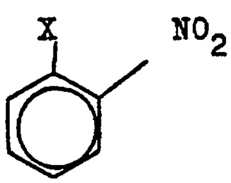

(II)

dans laquelle X a la signification qui lui a été donnée à propos du composé de formule I, en milieu alcalin, de manière à obtenir le composé répondant à la formule générale III:

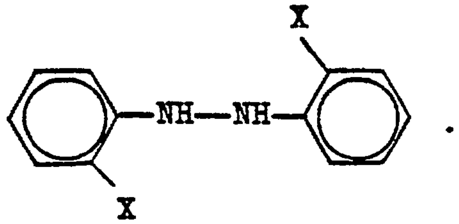

(III)

dans laquelle X a la signification qui lui a été donnée à propos de la formule I, puis on transpose le composé de formule III en le traitant pas un acide et on isole le produit obtenu sous la forme de la diamine libre, ou de son sel, répondant à la formule I.

7. Procédé selon la revendication 6 caractérisé en ce qu'on réduit le composé de formule II par de la poudre de zinc dans une solution d'un alcali.

8. Procédé selon l'une des revendications 6 et 7 caractérisé en ce qu'on transpose le composé de formule III au moyen d'acide chlorhydrique ou d'acide sulfurique.

9. Procédé selon l'une quelconque des revendications 6 à 8 caractérisé en ce que, dans la formule I, le symbole A correspond à l'équivalent de l'acide au moyen duquel le composé de formule III est transposé.

10. Application du composé selon la revendication 1 à la préparation de matières colorantes.

11. Application selon la revendication 10 à la préparation de colorants disazoïques ou de pigments disazoïques.

12. Application selon la revendication 10 comme composante de tétrazotation pour la préparation de colorants directs disazoïques.

13. Application selon la revendication 10 comme colorant base pour la préparation de colorants de développement par la technique des couleurs à la glace.